(19) Europäisches Patentamt
European Patent Office
Office européen des brevets

(11) Numéro de publication: **0 062 580**
**B1**

(12) **FASCICULE DE BREVET EUROPEEN**

(45) Date de publication du fascicule du brevet: **06.11.85**

(21) Numéro de dépôt: **82400590.4**

(22) Date de dépôt: **31.03.82**

(51) Int. Cl.$^4$: **C 07 D 471/04,**
C 07 D 215/38, A 61 K 31/47
// (C07D471/04, 235:00, 221:00)

(54) **Dérivés de l'imidazo(1,2-a)quinoléines.**

(30) Priorité: **03.04.81 GB 8110586**

(43) Date de publication de la demande:
**13.10.82 Bulletin 82/41**

(45) Mention de la délivrance du brevet:
**06.11.85 Bulletin 85/45**

(84) Etats contractants désignés:
**AT BE CH DE FR IT LI LU NL SE**

(56) Documents cités:
**FR-A-2 378 031**

(73) Titulaire: **ROUSSEL-UCLAF**
**35, boulevard des Invalides**
**F-75007 Paris (FR)**

(72) Inventeur: **Barnes, Alan Charles**
**12 St. Peter's Road**
**Cirencester, Glos (GB)**
Inventeur: **Robson, Peter Andrew**
**83 Queensfield Upper Stratton**
**Swindon Wiltshire (GB)**

(74) Mandataire: **Fritel, Hubert et al**
**ROUSSEL-UCLAF 111, route de Noisy Boîte**
**Postale no. 9**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 062 580 B1

**Description**

La présente invention concerne de nouvelles imidazo[1,2-a]quinoléines et leurs sels, leur préparation, leur application à titre de médicaments, les compositions les renfermant et les nouveaux intermédiaires obtenus.

La demande française FR—A—2378031 décrit des imidazoquinoléines possédant un groupement carboxylique en position 2 et présentant des propriétés antiallergiques et bronchodilalatrices.

L'invention a pour objet de nouvelles imidazo[1,2-a]quinoléines et leurs sels d'addition avec les acides, caractérisées en ce qu'elles répondent à la formule générale:

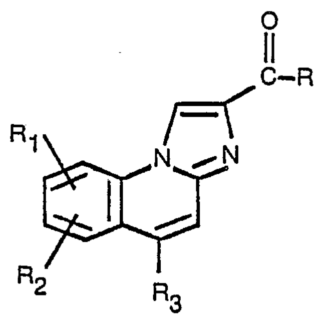

(I)

dans laquelle R représente un radical alcoyle renfermant de 1 à 8 atomes de carbone, un radical phényle éventuellement substitué par un ou plusieurs substituants choisis dans le groupe constitué par les halogènes, les radicaux alcoyles renfermant de 1 à 5 atomes de carbone et les radicaux alcoxy renfermant de 1 à 5 atomes de carbone, ou R représente un radical thiényl ou un radical pyridyl, $R_1$ et $R_2$, identiques ou différents, représentent un atome d'hydrogène, un atome d'halogène, un radical alcoyle renfermant de 1 à 8 atomes de carbone, un radical alcoxy renfermant de 1 à 8 atomes de carbone, un radical cyclohexyle, un radical phénoxy ou un radical nitro, $R_3$ représente un radical alcoxy ou alcoylthio renfermant de 1 à 8 atomes de carbone.

Dans la formule générale (I) et dans ce qui suit, le terme radical alcoyle renfermant de 1 à 8 atomes de carbone désigne, par exemple, un radical méthyle, éthyle, propyle, isopropyle, butyle, pentyle ou octyle; le terme radical alcoxy renfermant de 1 à 8 atomes de carbone désigne, par exemple, un radical méthoxy, éthoxy, propoxy, isopropoxy, butoxy, isobutoxy ou octyloxy; le terme radical alcoylthio renfermant de 1 à 8 atomes de carbone désigne, par exemple, un radical méthylthio, éthylthio, propylthio; le terme atome d'halogène désigne, par exemple, un atome de chlore, de fluor ou de brome.

Lorsque dans la formule générale (I), R représente un radical phényle substitué, le substituant est, de préférence, un atome de chlore ou de brome ou un radical méthyle ou méthoxy en position 4.

Les sels d'addition avec les acides minéraux ou organiques peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, alcanesulfoniques, tels que l'acide méthane sulfonique et arylsulfoniques, tels que l'acide benzène sulfonique.

Parmi les produits, objet de l'invention, on peut citer notamment les dérivés répondant à la formule (I) ci-dessus, ainsi que leurs sels, caractérisés en ce que dans ladite formule (I), R représente un radical méthyle ou phényle, $R_1$ et $R_2$, identiques ou différents, représentent un atome d'hydrogène, un atome de chlore, un radical méthyle, éthyle ou méthoxy, $R_3$ a la signification déjà indiquée.

Parmi les produits, objet de l'invention, on retient plus particulièrement les dérivés répondant à la formule (I) ci-dessus, ainsi que leurs sels, caractérisés en ce que dans ladite formule (I), R représente un radical phényle, $R_1$ représente un atome d'hydrogène, $R_2$ représente un atome d'hydrogène ou un radical alcoyle renfermant de 1 à 5 atomes de carbone et $R_3$ représente un radical alcoxy renfermant de 1 à 5 atomes de carbone.

Parmi ces derniers, on peut citer tout particulièrement:

— la (7-éthyl 5-méthoxy-imidazo[1,2-a]quinoléin-2-yl) phényl méthanone et son mésylate;

— la (5-méthoxy-imidazo[1,2-a]quinoléin-2-yl) phényl méthanone;

— la 5-isopropoxy-imidazo[1,2-a]quinoléin-2-yl) phényl méthanone;

— la (5-méthylthio-imidazo[1,2-a]quinoléin-2-yl) phényl méthanone.

L'invention a également pour objet un procédé de préparation des produits tels que définis par la formule (I) ci-dessus, ainsi que de leurs sels, caractérisé en ce que l'on fait réagir un produit de formule (II):

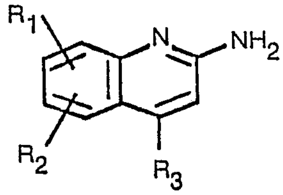

(II)

dans laquelle $R_1$, $R_2$ et $R_3$ ont la signification déjà indiquée, avec un produit de formule (III):

$$X—CH_2—CO—CO—R \qquad (III)$$

dans laquelle R a la signification déjà indiquée et X représente un atome d'halogène, pour obtenir un produit de formule (IV):

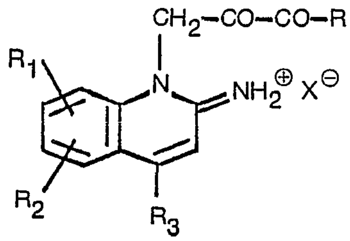

(IV)

dans laquelle R, $R_1$, $R_2$, $R_3$ et X ont la signification déjà indiquée, puis cyclise le produit de formule (IV) obtenu, pour former le produit de formule générale (I) que, le cas échéant, l'on salifie.

Dans les conditions préférentielles de mise en oeuvre de l'invention, le procédé de préparation ci-dessus décrit est caractérisé en ce que

— la réaction du produit de formule (II) avec le produit de formule (III) est effectuée au sein d'un solvant organique tel que le diméthoxyméthane;

— la cyclisation du produit de formule (IV) est effectuée par chauffage au sein d'un solvant organique tel que l'éthanol;

— la réaction du produit de formule (II) avec le produit de formule (III) est poursuivie jusqu'au produit de formule (I) sans isoler le produit de formule (IV).

Les produits de formule (I) présentent un caractère basique.

En général, la cyclisation du produit de formule (IV) conduit à un sel du produit de formule (I) avec l'acide HX formé. La base libre peut être obtenue par simple traitement par une base, par exemple un hydroxyde ou un carbonate alcalin.

On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

L'invention à également pour objet un procédé de préparation des produits tels que définis par la formule (I) ci-dessus, répondant à la formule ($I_a$):

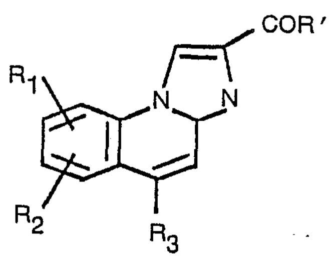

($I_a$)

dans laquelle R' représente un radical phényle éventuellement substitué par un ou plusieurs substituants choisis dans le groupe constitué par les halogènes, les radicaux alcoyles renfermant de 1 à 5 atomes de carbone et les radicaux alcoxy renfermant de 1 à 5 atomes de carbone, ou R' représente un radical thiényl ou un radical pyridil et $R_1$, $R_2$ et $R_3$ ont la signification déjà indiquée, ainsi que de leurs sels, caractérisé en ce que l'on traite un produit de formule (V):

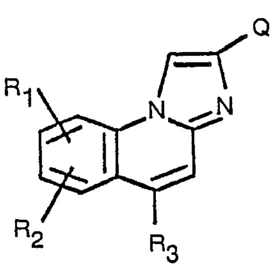

(V)

dans laquelle $R_1$, $R_2$ et $R_3$ ont la signification déjà indiquée et Q représente un groupement carboxylique estérifié, par un agent de réduction pour obtenir un produit de formule (VI):

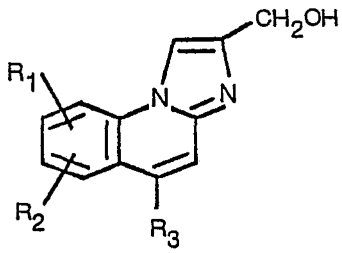

(VI)

dans laquelle $R_1$, $R_2$ et $R_3$ ont la signification déjà indiquée, que l'on traite par un agent d'oxydation, pour obtenir un produit de formule (VII):

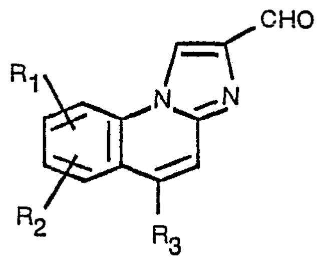

(VII)

dans laquelle $R_1$, $R_2$ et $R_3$ ont la signification déjà indiquée, que l'on fait réagir avec un réactif de formule (VIII):

$$R'—Y \quad (VIII)$$

dans laquelle R' a la signification déjà indiquée et Y représente un atome de chlore, de brome ou d'iode, pour obtenir un produit de formule (IX):

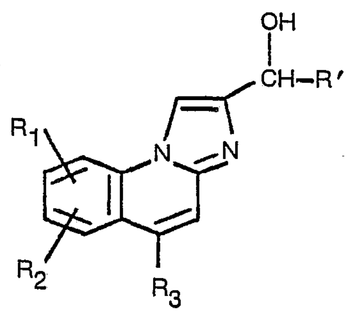

(IX)

dans laquelle $R_1$, $R_2$, $R_3$ et R' ont la signification déjà indiquée, que l'on traite par un agent d'oxydation, pour obtenir un produit de formule ($I_a$) que, le cas échéant, l'on salifie.

Dans les conditions préférentielles de mise en oeuvre de l'invention, le procédé de préparation ci-dessus décrit est caractérisé en ce que:

— Q, dans le produit de formule (V), représente un groupement alcoxycarbonyl, de préférence dans lequel alcoxy renferme de 1 à 3 atomes de carbone ou aralcoxycarbonyl;

— l'agent de réduction du produit de formule (V) est l'hydrure de lithium aluminium, le borohydrure de sodium ou de lithium, ou le chlorure d'aluminium;

— l'agent d'oxydation des produits de formule (VI) et (IX) est le dioxyde de manganèse, mais peut être également l'acide nitrique, le chlorure ferrique ou l'oxyde de chrome dans la pyridine; l'oxydation peut être également effectué selon la méthode d'Oppenauer ou par déshydrogénation sur un catalyseur à base de cuivre;

— la réaction du produit de formule (VII) avec le réactif de formule (VIII) est effectuée dans des conditions anhydres, dans un solvant organique, de préférence le tétrahydrofuranne.

La salification éventuelle des produits de formule ($I_a$) peut s'effectuer dans les conditions énoncées précédemment.

Les produits, objet de la précente invention, possèdent de très intéressantes propriétés pharmacologiques; ils sont doués notamment de remarquables propriétés anxiolytiques et d'une certaine activité hypnotique.

Ces propriétés sont illustrées plus loin dans la partie expérimentale.

Ces propriétés justifient l'utilisation des nouvelles imidazo[1,2-a]quinoléines de formule (I), ainsi que de leurs sels pharmaceutiquement acceptables, à titre de médicaments.

La présente invention a ainsi également pour objet l'application à titre de médicaments des nouvelles imidazo[1,2-a]quinoléines telles que définies par la formule générale (I) ainsi que de leurs sels d'addition avec les acides pharmaceutiquement acceptables.

Parmi les médicaments, objet de l'invention, on retient notamment, les médicaments caractérisés en ce qu'ils sont constitués par les nouvelles imidazo[1,2-a]quinoléines répondant à la formule (I) dans laquelle R représente un radical méthyle ou phényle, $R_1$ et $R_2$, identiques ou différents, représentent un atome d'hydrogène, un atome de chlore, un radical méthyle, éthyle ou méthoxy, $R_3$ a la signification déjà indiquée.

Parmi les médicaments, objet de l'invention on retient tout particulièrement ceux répondant à la formule (I), caractérisés en ce que R représente un radical phényle, $R_1$ représente un atome d'hydrogène, $R_2$ représente un atome d'hydrogène ou un radical alcoyle renfermant de 1 à 5 atomes de carbone et $R_3$ représente un radical alcoxy renfermant de 1 à 5 atomes de carbone.

Parmi les médicaments préférés de l'invention, on retient tout particulièrement:
— la (7-éthyl 5-méthoxy-imidazo[1,2-a]quinoléin-2-yl) phényl méthanone et son mésylate;
— la (5-méthoxy-imidazo[1,2-a]quinoléin-2-yl) phényl méthanone;
— la (5-isopropoxy-imidazo[1,2-a]quinoléin-2-yl) phényl méthanone;
— la (5-méthylthio-imidazo[1,2-a]quinoléin-2-yl) phényl méthanone.

Ces médicaments trouvent, par exemple, leur emploi dans le traitement des états anxieux, des anxiétés chroniques avec agitation, irritabilité, agressivité, des anxiétés avec insomnies et tensions musculaires, des angoisses.

La dose usuelle, variable selon le produit utilisé, le sujet traité et l'affection en cause peut être, par exemple, de 0,1 mg à 200 mg par jour, par voie orale.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparation injectables; elles sont préparées selon les méthodes usuelles. Le ou les principes actifs peuvent y être incorporés à des excipients habituellement employés dans dec compositions pharmaceutiques, tels que le talc, le gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (IV), utiles dans la préparation des produits de formule (I) sont des produits nouveaux et l'invention a ainsi également pour objet, à titre de produits industriels nouveaux et notamment à titre de produits intermédiaires nécessaires à la préparation des produits de formule (I) telle que définie ci-dessus, les produits de formule (IV), telle que définie ci-dessus.

Les produits de formules (VI), (VII) et (IX), que l'on peut regrouper dans la formule (X):

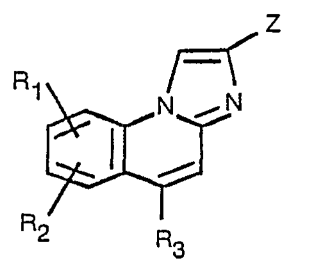

(X)

dans laquelle $R_1$, $R_2$ et $R_3$ ont la signification déjà indiquée et Z représente un groupement —CHO, $—CH_2OH$ ou

OH
|
—CH—R',

dans lequel R' a la signification déjà indiquée, utiles dans la préparation des produits de formule (I) sont également nouveaux et l'invention a ainsi enfin pour objet, à titre de produits industriels nouveaux et notamment à titre de produits intermédiaires nécessaires à la préparation des produits de formule (I) telle que définie ci-dessus, les produits de formule (X), telle que définie ci-dessus.

Les produits de formule (II), lorsqu'ils ne sont pas connus, peuvent être préparés par un procédé analogue à celui décrit dans la demande de brevet britannique n° 2377/77 déposée par la demanderesse ou dans le brevet britannique n° 1 542 778. Les produits de formule (II) peuvent également être préparés comme indiqué dans J.C.S. 1958 page 614 et suivantes ou dans Synthesis 1977 p. 500.

Les produits de formule (III) dans laquelle R représente un radical phényl peuvent être préparés comme indiqué dans Hel. Chim. Acta 1946, *29*, 1247. Les produits de formule (III) dans laquelle R représente un

radical méthyle peuvent être préparés comme indiqué dans le brevet US 2 821 555 (ceux pour lesquels R représente un radical alcoyle peuvent être préparés par un procédé analogue).

Les produits de formule (V) peuvent être préparés par la méthode décrite dans le brevet anglais 1 596 652 ou par des méthodes analogues.

Il va être donné maintenant, à titre non limitatif, des exemples de mise en oeuvre de l'invention.

Exemple 1

(7-éthyl 5-méthoxy-imidazo[1,2-a]quinoléin-2-yl) phényl méthanone.

On dissout 90 g de 2-amino 6-éthyl 4-méthoxyquinoléine dans 1,5 litre de diméthoxyméthane. On y ajoute 112,5 g de 3-bromo-1-phényl propan-1,2-dione et le mélange obtenu est maintenu pendant deux heures et demie sous agitation à température ambiante.

On filtre le précipité formé de bromhydrate de (6-éthyl 4-méthoxy[1-(3-phényl 2,3-dioxopropyl)]-quinoléin-2-iminium, le lave à l'éther, le met en suspension dans 1,25 litre d'éthanol et porte au reflux jusqu'à obtention d'une solution claire.

On refroidit à température ambiante et filtre les cristaux de sel formés et obtient 75 g de produit. On concentre les liqueurs-mères sous pression réduite, dilue à l'éther et filtre les cristaux formés.

On obtient ainsi 30 g d'un deuxième jet de produit.

On réunit les deux jets de produit et les agite avec un mélange de chloroforme et de solution aqueuse de carbonate de potassium.

La phase organique est séparée, lavée à l'eau, séchée, concentrée sous pression réduite et diluée à l'éthanol pour cristalliser.

On obtient ainsi en deux jets, 54,7 g de produit attendu.

F = 196—198°C.

*Spectre RMN* ($CDCl_3$)

Pic à 1,44 ppm (singulet, hydrogène en position 1).

Exemples 2 à 10

En utilisant une méthode analogue à celle utilisée dans l'exemple 1, on a obtenu les produits indiqués dans les Tables I et II ci-après.

Les composés montrent tous dans leur Spectre RMN ($CDCl_3$) un pic compris entre 1,2 et 1,7 ppm (singulet, hydrogène en position 1).

TABLEAU 1

| Exemple | R | $R_1$ | $R_2$ | $R_3$ | Sel | Rendement % | F°C | Spectre IR (KBr) $cm^{-1}$ |
|---|---|---|---|---|---|---|---|---|
| 1 | 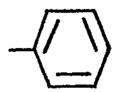 | H | 7–$C_2H_5$ | $OCH_3$ | – | 37 | 196–8 | 3140,1645,1606 |
| 2 | 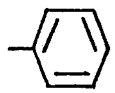 | H | H | $SCH_3$ | – | 25 | 209 | 3125,1645,1602 |
| 3 | 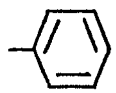 | 8–$OCH_3$ | H | $OCH_3$ | – | 16 | 180–2 | 1640 |
| 4 | 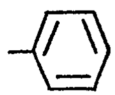 | H | H | $OCH_3$ | – | 12 | 190–1 | 3140,1650 |
| 5 | 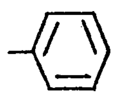 | H | 7–$C_2H_5$ | $OCH_3$ | $CH_3SO_3H$ | | 228–30 | 3075,1665,1644,1601 |
| 6 | $-CH_3$ | H | 7–$C_2H_5$ | $OCH_3$ | – | 30 | 148 | 3140,1690 |

TABLEAU 1 (Suite)

| Exemple | R | $R_1$ | $R_2$ | $R_3$ | Sel | Rendement % | F°C | Spectre IR (KBr) $cm^{-1}$ |
|---|---|---|---|---|---|---|---|---|
| 7 | 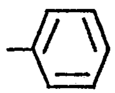 | H | 7–Cl | $OCH_3$ | – | 12 | 228–34 | 3140,1650 |
| 8 | 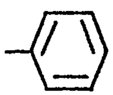 | H | H | O*i*Pr | – | 6 | 162 | 3140,3000,2900,1640 |
| 9 | 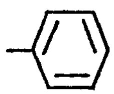 | H | H | $OC_2H_5$ | – | 14 | 210 | 3140,1640 |
| 10 | 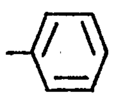 | H | H | OBu | – | 20 | 159 | 3360,3090,2980, 1640,1620 |

TABLEAU 2

| Exemple | Formule brute | Poids moléculaire | Calculé/Trouvé | | | |
|---|---|---|---|---|---|---|
| | | | C | H | N | X |
| 1 | $C_{21}H_{18}N_2O_2$ | 330.37 | 76.33<br>76.5 | 5.50<br>5.5 | 8.48<br>8.5 | |
| 2 | $C_{19}H_{14}N_2OS$ | 318.39 | 71.67<br>71.8 | 4.43<br>4.5 | 8.80<br>8.8 | |
| 3 | $C_{20}H_{16}N_2O_3$ | 332.36 | 72.28<br>72.2 | 4.85<br>4.9 | 8.43<br>8.4 | |
| 4 | $C_{19}H_{14}N_2O_2$ | 302.33 | 75.48<br>75.3 | 4.67<br>4.7 | 9.26<br>9.2 | |
| 5 | $C_{22}H_{22}N_2O_5S$ | 426.49 | 61.95<br>62.2 | 5.20<br>5.2 | 6.57<br>6.7 | 7.51 (S)<br>7.7 |
| 6 | $C_{16}H_{16}N_2O_2$ | 268.31 | 71.62<br>71.7 | 6.01<br>6.1 | 10.44<br>10.6 | |
| 7 | $C_{19}H_{13}ClN_2O_3$ | 336.7 | 67.76<br>67.5 | 3.89<br>4.0 | 8.32<br>8.4 | 10.53(Cl)<br>10.7 |
| 8 | $C_{21}H_{18}N_2O_2$ | 330.36 | 76.35<br>76.4 | 5.49<br>5.6 | 8.48<br>8.4 | |
| 9 | $C_{20}H_{16}N_2O_2$ | 316.34 | 75.93<br>75.9 | 5.10<br>5.2 | 8.85<br>8.8 | |
| 10 | $C_{22}H_{20}N_2O_2$ | 344.39 | 76.72<br>76.5 | 5.85<br>5.9 | 8.13<br>8.1 | |

Exemple 11

a) *5-méthoxy imidazo[1,2-a]quinoléin-2-méthanol*

On agite à température ambiante sous atmosphère inerte, une suspension de 19,71 g de 5-méthoxy imidazo[1,2-a]quinoléin-2-carboxylate d'éthyle dans 350 cm3 de tétrahydrofuranne, puis ajoute par portions en trois heures, 3,33 g d'hydrure de lithium aluminium.

On agite ensuite le mélange pendant quatre heures, détruit l'excès d'hydrure par addition de tétrahydrofuranne humide puis par addition d'eau, puis verse le mélange dans 1,5 litre d'eau.

On extrait par le mélange chloroforme-méthanol, sépare la phase organique et la filtre.

On réextrait la phase aqueuse avec le mélange chloroforme-méthanol, joint les phases organiques, les sèche et évapore le solvant. On triture le résidu dans l'acétate d'éthyle, filtre, sèche et obtient 14,87 g de produit attendu. F = 173—175°C.

b) *5-méthoxy imidazo[1,2-a]quinoléin-2-carboxyaldéhyde*

On agite une solution de 14,6 g du produit obtenu au Stade a) dans 700 cm3 de chloroforme, puis ajoute 60 g de dioxyde de manganèse activé. On agite à température ambiante pendant vingt-huit heures, filtre le mélange et lave le filtre au chloroforme. On évapore le filtrat à sec et obtient après recristallisation dans l'acétate d'éthyle 10,9 g de produit attendu. F = 195—196°C.

c) *(4-éthyl phényl) (5-méthoxy imidazo[1,2-a]quinoléin-2-yl)méthanol.*

On ajoute gotte à goutte 2,78 g de parabromoéthyl benzène dans une suspension agitée de 0,36 g de tournure de magnésium dans 25 cm3 de tétrahydrofuranne, en présence d'un cristal d'iode, le mélange étant tiédi ou refroidi selon nécessité pour former le réactif. La solution est ensuite ajoutée à l'aide d'une seringue dans une suspension de 2,26 g de 5-méthoxy imidazo[1,2-a]quinoléin-2-carboxaldéhyde dans 50 cm3 de tétrahydrofuranne. Après avoir agité à température ambiante pendant une heure, le mélange réactionnel est versé dans 200 cm3 d'eau et extrait au chloroforme. La phase organique est séchée et évaporée à sec sous pression réduite. Le résidu est purifié par chromatographie sur silice en éluant au mélange chloroforme-méthanol (95—5). On obtient 2,12 g de produit attendu sous forme amorphe.

d) *(4-éthyl phényl) (5-méthoxyimidazo[1,2-a]quinoléin-2-yl) méthanone.*

On agite 8 g de dioxyde de manganèse activé à une solution de 1,99 g de produit obtenu au Stade c) dans 100 cm3 de chloroforme, puis le mélange obtenu est agité vigoureusement à température ambiante pendant seize heures.

On filtre le mélange, rince l'insoluble au chloroforme, on évapore le solvant sous pression réduite. On recristallise le résidu dans l'acétate d'éthyle et obtient 1,32 g de produit attendu. F = 147—148°C.

*Spectre IR* (KBr)

Absorptions à 3130—1630—1600 $cm^{-1}$

*Analyse*: $C_{21}H_{18}N_2O_2$ (330, 38)

| | | | |
|---|---|---|---|
| Calculé: | C% 76,34 | H% 5,49 | N% 8,48 |
| Trouvé: | 76,3 | 5,6 | 8,5. |

Exemples 12 à 33

Les produits ont été préparés comme indiqué dans les Tableaux III et IV ci-après.

La méthode utilisée pour la préparation a été soit: celle de l'exemple 1 (Méthode a) soit celle de l'exemple 11 (Méthode b).

TABLEAU 3

| Ex. | R | $R_1$ | $R_2$ | $R_3$ | Rdt % | Méthode | F°C | Spectre IR (KBr) $cm^{-1}$ |
|---|---|---|---|---|---|---|---|---|
| 12 | 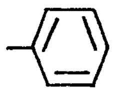 | H | H | OPr | 13 | A | 179 | 3120, 1640 |
| 13 | 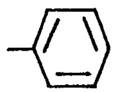 | H | 7–$CH_3$ | $OCH_3$ | 30 | A | 218–20 | 3120, 1630, 1600 |
| 14 | 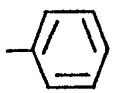 | H | 7–$OCH_3$ | $OCH_3$ | 20 | A | 233–5 | 3130, 1640, 1600 |
| 15 | 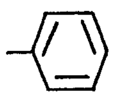 | H | 7–*i*Pr | $OCH_3$ | 23 | A | 187–3 | 3110, 1630 |
| 16 | 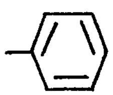 | H | 7–Bu | $OCH_3$ | 26 | A | 165–7 | 3120, 1630 |
| 17 | 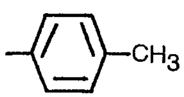 | H | H | $OCH_3$ | 35 | B | 212–3 | 3120, 1630, 1600 |
| 18 | 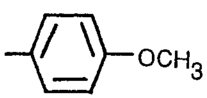 | H | H | $OCH_3$ | 31 | B | 170–1 | 3120, 1630, 1600 |

TABLEAU 3 (Suite)

| Ex. | R | $R_1$ | $R_2$ | $R_3$ | Rdt % | Méthode | F°C | Spectre IR (KBr) $cm^{-1}$ |
|---|---|---|---|---|---|---|---|---|
| 19 | 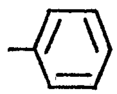 | 9–$OCH_3$ | H | $OCH_3$ | 24.5 | A | 189–91 | 3180, 1640 |
| 20 | 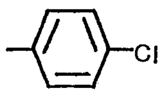 | H | H | $OCH_3$ | 25 | B | 245–6 | 3130, 1635, 1585 |
| 21 | 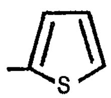 | H | H | $OCH_3$ | 36 | B | 257–9 | 3100, 1630, 1610 |
| 22 | 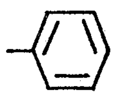 | 8–Cl | H | $OCH_3$ | 30 | A | 211–13 | 3100, 1640, 1600 |
| 23 | 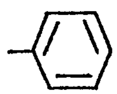 | 9–Cl | H | $OCH_3$ | 43 | A | 149–50 | 3200, 1630, 1600 |
| 24 | 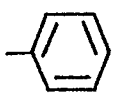 | 8–$CH_3$ | H | $OCH_3$ | 21.6 | A | 192–3 | 3130, 1640, 1600 |
| 25 | 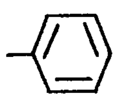 | H | 6–$CH_3$ | $OCH_3$ | 9.4 | A | 209–10 | 3130, 1640, 1600 |

| Ex. | R | $R_1$ | $R_2$ | $R_3$ | Rdt % | Méthode | F°C | Spectre IR (KBr) $cm^{-1}$ |
|---|---|---|---|---|---|---|---|---|
| 26 | 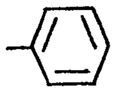 | H | 7 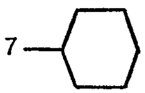 | $OCH_3$ | 70 | A | 227 | 3120, 1640 |
| 27 | 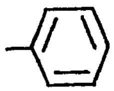 | H | 7–OPh | $OCH_3$ | | A | 210–12 | 3120, 1640 |
| 28 | 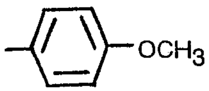 | H | 7–$C_2H_5$ | $OCH_3$ | 13 | B | 172–3 | 3120, 1635, 1600 |
| 29 | 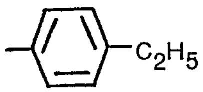 | H | 7–$C_2H_5$ | $OCH_3$ | 24 | B | 170–1 | 3120, 1640, 1605 |
| 30 | 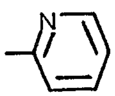 | H | 7–$C_2H_5$ | $OCH_3$ | 15 | B | 185–6 | 3180, 1650, 1630 |
| 31 | 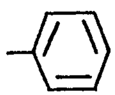 | H | 7–F | $OCH_3$ | 19 | A | 234–5 | 3160, 1630, 1600 |
| 32 | 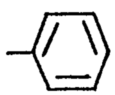 | H | 7–*n*Pr | $OCH_3$ | 30 | A | 185–6 | 3120, 1640, 1600 |
| 33 | 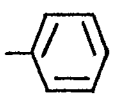 | 8–$CH_3$ | 7–$CH_3$ | $OCH_3$ | 28 | A | 244–5 | 3120, 1630, 1590 |

TABLEAU 4

| Ex. | Formule brute | Poids Moléc. | Calculé/Trouvé C | H | N | X |
|---|---|---|---|---|---|---|
| 12 | $C_{21}H_{18}N_2O_2$ | 330.38 | 76.34<br>76.4 | 5.49<br>5.6 | 8.48<br>8.5 | |
| 13 | $C_{20}H_{16}H_2O_2$ | 316.36 | 75.93<br>75.6 | 5.10<br>5.2 | 8.85<br>8.8 | |
| 14 | $C_{20}H_{16}N_2O_3$ | 332.36 | 72.28<br>72.5 | 4.85<br>4.9 | 8.43<br>8.4 | |
| 15 | $C_{22}H_{20}N_2O_2$ | 344.39 | 76.72<br>76.9 | 5.85<br>5.9 | 8.13<br>8.2 | |
| 16 | $C_{23}H_{22}N_2O_2$ | 358.45 | 77.07<br>77.3 | 6.19<br>6.2 | 7.81<br>7.8 | |
| 17 | $C_{20}H_{16}N_2O_2$ | 316.36 | 75.93<br>75.9 | 5.10<br>5.15 | 8.85<br>8.8 | |
| 18 | $C_{20}H_{16}N_2O_3$ | 332.36 | 72.28<br>72.2 | 4.85<br>4.9 | 8.43<br>8.4 | |
| 19 | $C_{20}H_{16}N_2O_3$ | 332.36 | 72.28<br>72.1 | 4.85<br>4.9 | 8.43<br>8.4 | |
| 20 | $C_{19}H_{13}ClN_2O_2$ | 336.77 | 67.76<br>67.75 | 3.89<br>4.0 | 8.32<br>8.3 | 10.53(Cl)<br>10.8 |
| 21 | $C_{17}H_{12}N_2O_2S$ | 308.36 | 66.22<br>66.2 | 3.92<br>4.0 | 9.08<br>9.1 | 10.40(S)<br>10.4 |
| 22 | $C_{19}H_{13}ClN_2O_2$ | 336.77 | 67.76<br>67.8 | 3.89<br>3.95 | 8.32<br>8.3 | 10.53(Cl)<br>10.6 |
| 23 | $C_{19}H_{13}ClN_2O_2$ | 336.77 | 67.76<br>67.8 | 3.89<br>3.9 | 8.32<br>8.3 | 10.53(Cl)<br>10.6 |
| 24 | $C_{20}H_{16}N_2O_2$ | 316.36 | 75.93<br>75.7 | 5.10<br>5.2 | 8.85<br>8.9 | |
| 25 | $C_{20}H_{16}N_2O_2$ | 316.34 | 75.93<br>75.65 | 5.10<br>5.2 | 8.85<br>8.9 | |
| 26 | $C_{25}H_{24}N_2O_2$ | 384.45 | 78.10<br>78.2 | 6.39<br>6.4 | 7.29<br>7.3 | |
| 27 | $C_{25}N_{18}N_2O_3$ | 394.43 | 76.13<br>75.9 | 4.60<br>4.7 | 7.10<br>7.0 | |
| 28 | $C_{22}H_{20}N_2O_3$ | 360.42 | 73.32<br>73.3 | 5.59<br>5.65 | 7.77<br>7.8 | |
| 29 | $C_{23}H_{22}N_2O_2$ | 358.45 | 77.07<br>77.3 | 6.19<br>6.2 | 7.82<br>7.9 | |

TABLEAU 4 (Suite)

| Ex. | Formule brute | Poids Moléc. | Calculé/Trouvé C | H | N | X |
|---|---|---|---|---|---|---|
| 30 | $C_{20}H_{17}N_3O_2$ | 331.38 | 72.49<br>72.6 | 5.17<br>5.2 | 12.68<br>12.8 | |
| 31 | $C_{19}H_{13}N_2O_2F$ | 320.32 | 71.24<br>71.1 | 4.09<br>4.1 | 8.74<br>8.7 | 5.93(F)<br>5.8 |
| 32 | $C_{22}H_{20}N_2O_2$ | 344.39 | 76.72<br>76.8 | 5.85<br>5.9 | 8.13<br>8.2 | |
| 33 | $C_{21}H_{18}N_2O_2$ | 330.40 | 76.34<br>76.2 | 5.49<br>5.55 | 8.48<br>8.4 | |

ETUDE PHARMACOLOGIQUE DES COMPOSES SELON L'INVENTION:

a) *Biochimie*

L'affinité des composés pour le récepteur des benzodiazépines a été déterminée en utilisant le radioligant [$^3$H] flunitrazépam selon une méthode dérivée de celle qui a été décrite par Squires et Braestrup dans Nature, 1977, *266*, p. 732.

Les valeurs figurant dans le tableau ci-après constituent les concentrations nanomolaires de produits testés qui inhibent d'une valeur de 50% (CI50nM) la fixation spécifique de [$^3$H] flunitrazépam à la concentration de 0,6 nM dana une préparation de membrane de cerveau antérieur de rat.

| Produit de l'exemple | $CI_{50}nM$ |
|---|---|
| 1 | 14 |
| 2 | 500 |
| 3 | 5000 |
| 4 | 27 |
| 5 | – |
| 6 | 1300 |
| 7 | 600 |
| 8 | 470 |
| 9 | 540 |
| 10 | 1000 |
| 11 | 67 |
| 12 | 1000 |
| 13 | 21 |
| 14 | 51 |
| 15 | 25 |
| 16 | 57 |
| 17 | 19 |
| 18 | 37 |
| 19 | 630 |
| 20 | 96 |
| 21 | 24 |
| 22 | 340 |
| 23 | 132 |
| 24 | 290 |
| 25 | 11 |
| 26 | 10000 |
| 27 | 210 |
| 28 | 30 |
| 29 | 51 |
| 30 | 92 |
| 31 | 74 |
| 32 | – |
| 33 | – |

b) *Etude de l'activité anxiolytique*

L'étude de l'activité anxiolytique a été effectuée en utilisant la méthode "lick shock" de Vogel et al. (Psychopharmacologia. 1971, *21*, 1). Les valeurs indiquées dans le tableau sont les doses effectives minimales (mg/kg per os) auxquelles on observe un accroissement du nombre des chocs reçus, par rapport à un témoin.

| Produit de l'exemple | DEM mg/kg |
|---|---|
| 1 | 20 |
| 4 | 25 |
| 6 | 20 |
| 13 | 25 |
| 15 | 10 |
| 16 | 25 |

Exemples de compositions pharmaceutiques.

1) On a préparé des comprimés répondant à la formule:

— (7-éthyl 5-méthoxy-imidazo[1,2-a]quinoléin-2-yl) phényl méthanone 20 mg
— Excipient q.s. pour un comprimé terminé à 150 mg.

(Détail de l'excipient: lactose, amidon, talc, stéarate de magnésium).

2) On a préparé des comprimés répondant à la formule:

— (5-méthoxy-imidazo[1,2-a]quinoléin-2-yl) phényl méthanone 20 mg
— Excipient q.s. pour un comprimé terminé à 150 mg.

(Détail de l'excipient: lactose, amidon, talc, stéarate de magnésium).

**Revendications pour les Etats contractants: BE CH DE FR IT LI LU NL SE**

1. Imidazo[1,2-a]quinoléine et leurs sels, caractérisées en ce qu'elles répondent à la formule générale:

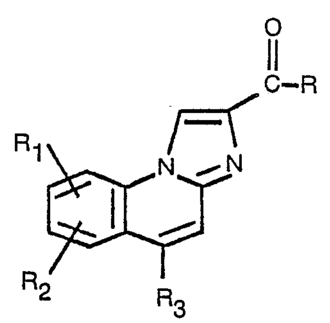

(I)

dans laquelle R représente un radical alcoyle renfermant de 1 à 8 atomes de carbone, un radical phényle éventuellement substitué par un ou plusieurs substituants choisis dans le groupe constitué par les halogènes, les radicaux alcoyles renfermant de 1 à 5 atomes de carbone et les radicayx alcoxy renfermant de 1 à 5 atomes de carbone, ou R représente un radical thiényl ou un radical pyridyl, $R_1$ et $R_2$, identiques ou différents, représentent un atome d'hydrogène, un atome d'halogène, un radical alcoyle renfermant de 1 à 8 atomes de carbone, un radical alcoxy renfermant de 1 à 8 atomes de carbone, un radical cyclohexyle, un radical phénoxy ou un radical nitro, $R_3$ représente un radical alcoxy ou alcoylthio renfermant de 1 à 8 atomes de carbone.

2. Dérivés répondant à la formule (I) de la revendication 1, ainsi que leurs sels, caractérisés en ce que dans ladite formule (I) R représente un radical méthyle ou phényle $R_1$ et $R_2$, identiques ou différents, représentent un atome d'hydrogène, un atome de chlore, un radical méthyle, éthyle ou méthoxy, $R_3$ a la signification déjà indiquée.

3. Dérivés répondant à la formule (I) de la revendication 1, ainsi que leurs sels, caractérisés en ce que dans ladite formule (I) R représente un radical phényle, $R_1$ représente un atome d'hydrogène, $R_2$ représente un atome d'hydrogène ou un radical alcoyle renfermant de 1 à 5 atomes de carbone et $R_3$ représente un radical alcoxy renfermant de 1 à 5 atomes de carbone.

4. Imidazo[1,2-a]quinoléine répondant à la formule (I) de la revendication 1 dont le nom suit: la (7-éthyl 5-méthoxy-imidazo[1,2-a]quinoléin-2-yl)phényl méthanone et son mésylate.

5. Imidazo[1,2-a]quinoléine répondant à la formule (I) de la revendication 1 dont le nom suit: la (5-méthoxy-imidazo[1,2-a]quinoléin-2-yl)phényl méthanone.

6. Imidazo[1,2-a]quinoléine répondant à la formule (I) de la revendication 1 dont le nom suit: la (5-isopropoxy-imidazo[1,2-a]quinoléin-2-yl)phényl méthanone.

7. Imidazo[1,2-a]quinoléine répondant à la formule (I) de la revendication 1 dont le nom suit: la (5-méthylthio-imidazo[1,2-a]quinoléin-2-yl) phényl méthanone.

8. Procédé de préparation des produits répondant à la formule générale (I), ainsi que de leurs sels, caractérisé en ce que l'on fait réagir un produit de formule (II):

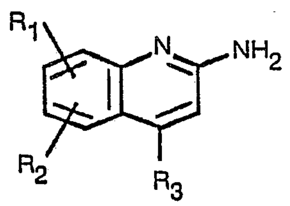

(II)

dans laquelle $R_1$, $R_2$ et $R_3$ ont la signification déjà indiquée, avec un produit de formule (III):

$$X—CH_2—CO—CO—R \qquad (III)$$

dans laquelle R a la signification déjà indiquée et X représente un atome d'halogène, pour obtenir un produit de formule (IV):

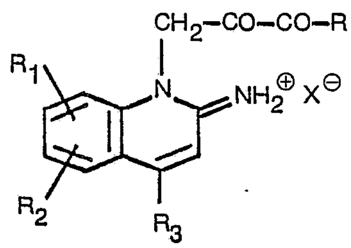

(IV)

dans laquelle R, $R_1$, $R_2$, $R_3$ et X ont la signification déjà indiquée, puis cyclise le produit de formule (IV) obtenu, pour former le produit de formule générale (I) que, le cas échéant, l'on salifie.

9. Procédé selon la revendication 8, caractérisé en ce que:

— la réaction du produit de formule (II) avec le produit de formule (III) est effectuée au sein d'un solvant organique tel que le diméthoxyméthane;

— la cyclisation du produit de formule (IV) est effectuée par chauffage au sein d'un solvant organique tel que l'éthanol;

— la réaction du produit de formule (II) avec le produit de formule (III) est poursuivie jusqu'au produit de formule (I) sans isoler le produit de formule (IV).

10. Procédé de préparation des produits tels que définis par la formule (I) de la revendication 1, répondant à la formule ($I_a$):

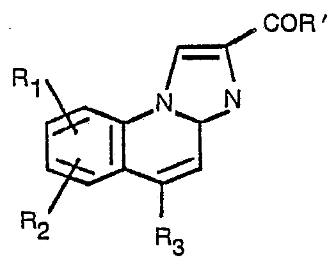

($I_a$)

dans laquelle R' représente un radical phényle éventuellement substitué par un ou plusieurs substituants choisis dans le groupe constitué par les halogènes, les radicaux alcoyles renfermant de 1 à 5 atomes de carbone et les radicaux alcoxy renfermant de 1 à 5 atomes de carbone, ou R' représente un radical thiényl ou un radical pyridil et $R_1$, $R_2$ et $R_3$ ont la signification déjà indiquée, ainsi que de leurs sels, caractérisé en ce que l'on traite un produit de formule (V):

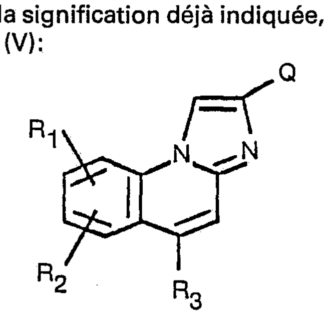

(V)

dans laquelle $R_1$, $R_2$ et $R_3$ ont la signification déjà indiquée et Q représente un groupement carboxylique estérifié, par un agent de réduction pour obtenir un produit de formule (VI):

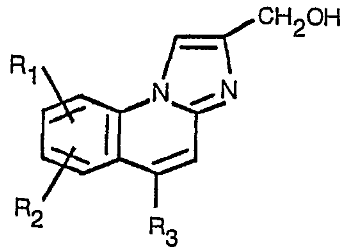

(VI)

dans laquelle $R_1$, $R_2$ et $R_3$ ont la signification déjà indiquée, que l'on traite par un agent d'oxydation, pour obtenir un produit de formule (VII):

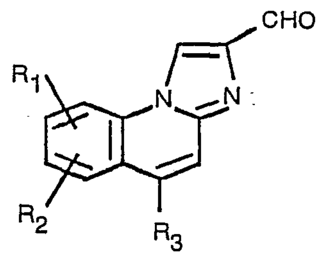

(VII)

dans laquelle $R_1$, $R_2$ et $R_3$ ont la signification déjà indiquée, que l'on fait réagir avec un réactif de formule (VIII):

$$R'—Y \qquad (VIII)$$

dans laquelle R' a la signification déjà indiquée et Y représente un atome de chlore, de brome ou d'iode, pour obtenir un produit de formule (IX):

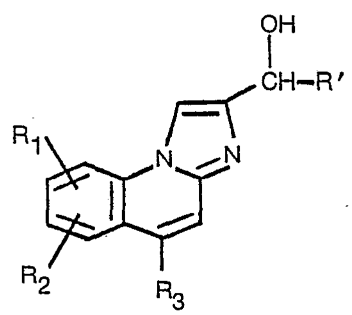

(IX)

dans laquelle $R_1$, $R_2$, $R_3$ et R' ont la signification déjà indiquée, que l'on traite par un agent d'oxydation, pour obtenir un produit de formule ($I_a$) que, le cas échéant, l'on salifie.

11. Procédé selon la revendication 10, caractérisé en ce que:

— Q, dans le produit de formule (V), représente un groupement alcoxycarbonyl, de préférence dans lequel alcoxy renferme de 1 à 3 atomes de carbone, ou aralcoxycarbonyl;

— l'agent de réduction du produit de formule (V) est l'hydrure de lithium aluminium;

— l'agent d'oxydation des produits de formules (VI) et (IX) est le dioxyde de manganèse;

— la réaction du produit de formule (VII) avec le réactif de formule (VIII) est effectuée dans des conditions anhydres, dans un solvant organique, de préférence le tétrahydrofuranne.

12. Médicaments, caractérisés en ce qu'ils sont constitués par les imidazo[1,2-a]quinoléines telles que définies à la revendication 1, ainsi que par leurs sels d'addition avec les acides pharmaceutiquement acceptables.

13. Médicaments, caractérisés en ce qu'ils sont constitués par les imidazo[1,2-a]quinoléines telles que définies à la revendication 2 ou 3, ainsi que par leurs sels d'addition avec les acides pharmaceutiquement acceptables.

14. Médicaments, caractérisés en ce qu'ils sont constitués par les dérivés tels que définis à l'une quelconque des revendications 4, 5, 6 ou 7, ainsi que par les sels d'addition avec les acides pharmaceutiquement acceptables desdits dérivés.

15. Compositions pharmaceutiques, caractérisées en ce qu'elles renferment à titre de principe actif, l'un au moins des médicaments tels que définis à l'une quelconque des revendications 12, 13 ou 14.

16. A titre de produits industriels, les produits de formule (IV):

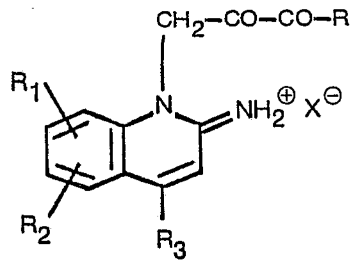

(IV)

telle que définie à la revendication 8.

17. A titre de produits industriels, les produits de formule (X):

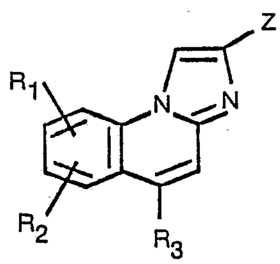

(X)

dans laquelle $R_1$, $R_2$ et $R_3$ sont définis comme à la revendication 1 et Z représente un groupement —CH O, —$CH_2$OH ou

$$-\underset{}{\overset{OH}{\overset{|}{C}H}}-R',$$

dans lequel R' est défini comme à a revendication 10.

**Revendications pour l'Etats contractant: AT**

1. Procédé pour préparer les imidazo[1,2-a]quinoléines répondant à la formule générale:

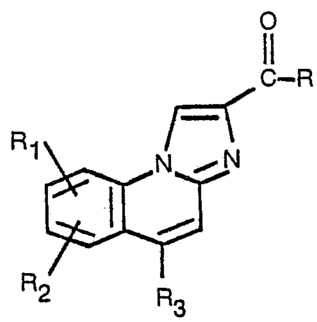

(I)

dans laquelle R représente un radical alcoyle renfermant de 1 à 8 atomes de carbone, un radical phényle éventuellement substitué par un ou plusieurs substituants choisis dans le groupe constitué par les halogènes, les radicaux alcoyles renfermant de 1 à 5 atomes de carbone et les radicayx alcoxy renfermant de 1 à 5 atomes de carbone, ou R représente un radical thiényl ou un radical pyridyl, $R_1$ et $R_2$, identiques ou différents, représentent un atome d'hydrogène, un atome d'halogène, un radical alcoyle renfermant de 1 à 8 atomes de carbone, un radical alcoxy renfermant de 1 à 8 atomes de carbone, un radical cyclohexyle, un radical phénoxy ou un radical nitro, $R_3$ représente un radical alcoxy ou alcoylthio renfermant de 1 à 8 atomes de carbone, ainsi que leurs sels, caractérisé en ce que l'on fait réagir un produit de formule (II):

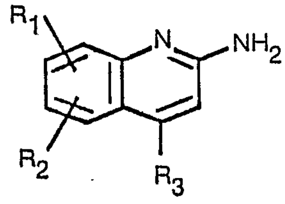

(II)

dans laquelle $R_1$, $R_2$ et $R_3$ ont la signification déjà indiquée, avec un produit de formule (III):

$$X—CH_2—CO—CO—R \quad (III)$$

dans laquelle R a la signification déjà indiquée et X représente un atome d'halogène, pour obtenir un produit de formule (IV):

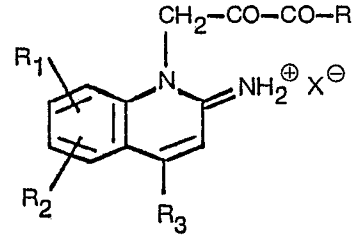

(IV)

dans laquelle R, $R_1$, $R_2$, $R_3$ et X ont la signification déjà indiquée, puis cyclise le produit de formule (IV) obtenu, pour former le produit de formule générale (I) que, le cas échéant, l'on salifie.

2. Procédé selon la revendication 1, caractérisé en ce que:

— la réaction du produit de formule (II) avec le produit de formule (III) est effectuée au sein d'un solvant organique tel que le diméthoxyméthane;

— la cyclisation du produit de formule (IV) est effectuée par chauffage au sein d'un solvant organique tel que l'éthanol;

— la réaction du produit de formule (II) avec le produit de formule (III) est poursuivie jusqu'au produit de formule (I) sans isoler le produit de formule (IV).

3. Procédé de préparation des produits tels que définis par la formule (I) de la revendication 1, répondant à la formule ($I_a$):

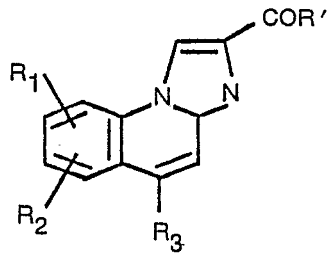

($I_a$)

dans laquelle R' représente un radical phényle éventuellement substitué par un ou plusieurs substituants choisis dans le groupe constitué par les halogènes, les radicaux alcoyles renfermant de 1 à 5 atomes de carbone et les radicaux alcoxy renfermant de 1 à 5 atomes de carbone, ou R' représente un radical thiényl ou un radical pyridil et $R_1$, $R_2$ et $R_3$ ont la signification déjà indiquée, ainsi que de leurs sels, caractérisé en ce que l'on traite un produit de formule (V):

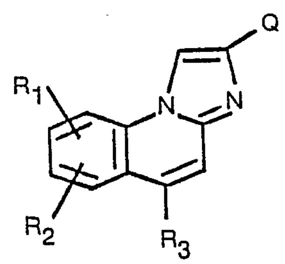

(V)

dans laquelle $R_1$, $R_2$ et $R_3$ ont la signification déjà indiquée et Q représente un groupement carboxylique estérifié, par un agent de réduction pour obtenir un produit de formule (VI):

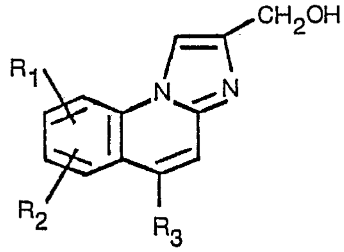

(VI)

dans laquelle $R_1$, $R_2$ et $R_3$ ont la signification déjà indiquée, que l'on traite par un agent d'oxydation, pour obtenir un produit de formule (VII):

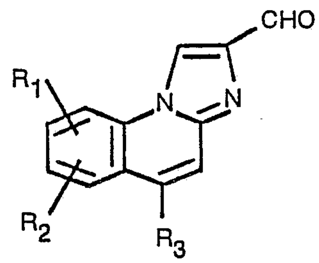

(VII)

dans laquelle $R_1$, $R_2$ et $R_3$ ont la signification déjà indiquée, que l'on fait réagir avec un réactif de formule (VIII):

$$R'—Y \quad (VIII)$$

dans laquelle R' a la signification déjà indiquée et Y représente un atome de chlore, de brome ou d'iode, pour obtenir un produit de formule (IX):

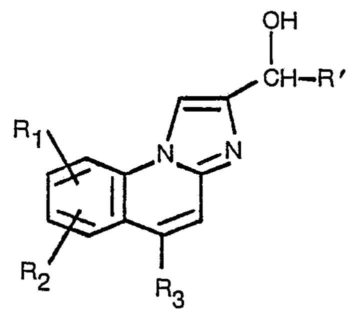

(IX)

dans laquelle $R_1$, $R_2$, $R_3$ et R' ont la signification déjà indiquée, que l'on traite par un agent d'oxydation, pour obtenir un produit de formule ($I_a$) que, le cas échéant, l'on salifie.

4. Procédé selon la revendication 3, caractérisé en ce que:

— Q, dans le produit de formule (V), représente un groupement alcoxycarbonyl, de préférence dans lequel alcoxy renferme de 1 à 3 atomes de carbone, ou aralcoxycarbonyl;

— l'agent de réduction du produit de formule (V) est l'hydrure de lithium aluminium;

— l'agent d'oxydation des produits de formules (VI) et (IX) est le dioxyde de manganèse;

— la réaction du produit de formule (VII) avec le réactif de formule (VIII) est effectuée dans des conditions anhydres, dans un solvant organique, de préférence le tétrahydrofuranne.

5. Procédé selon l'une quelconque des revendications 1 à 4, caractérisé en ce que l'on prépare des dérivés de formule I dans laquelle R représente un radical méthyle ou phényle, $R_1$ et $R_2$, identiques ou différents, représentent un atome d'hydrogène, un atome de chlore, un radical méthyle, éthyle ou méthoxy, $R_3$ a la signification déjà indiquée.

6. Procédé selon l'une quelconque des revendications 1 à 4, caractérisé en ce que l'on prépare des dérivés de formule I dans laquelle R représente un radical phényle, $R_1$ représente un atome d'hydrogène, $R_2$ représente un atome d'hydrogène ou un radical alcoyle renfermant de 1 à 5 atomes de carbone et $R_3$ représente un radical alcoxy renfermant de 1 à 5 atomes de carbone.

7. Procédé selon l'une quelconque des revendications 1 à 4, caractérisé en ce que l'on prépare des dérivés de formule I dans laquelle l'un quelconque des dérivés de formule I dont les noms suivent:

— La (7-éthyl 5-méthoxy-imidazo[1,2-a]quinoléin-2-yl) phényl méthanone et son mésylate.

— La (5-méthoxy-imidazo[1,2-a]quinoléin-2-yl) phényl méthanone.

— La (5-isopropoxy-imidazo[1,2-a]quinoléin-2-yl) phényl méthanone.

— La (5-méthylthio-imidazo[1,2-a]quinoléin-2-yl) phényl méthanone.

**Claims for the Contracting States: BE CH DE FR IT LI LU NL SE**

1. Imidazo[1,2-a]quinolines and their salts, characterized in that they answer to the general formula:

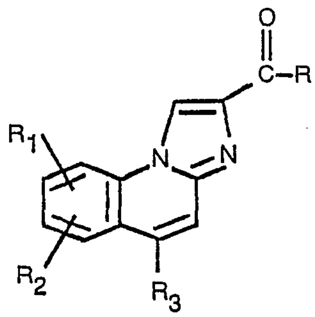

(I)

in which R represents an alkyl radical containing from 1 to 8 carbon atoms, a phenyl radical possibly substituted by one or more substituents chosen from the group constituted by the halogens, the alkyl radicals containing from 1 to 5 carbon atoms and the alkoxy radicals containing from 1 to 5 carbon atoms, or R represents a thienyl radical or a pyridyl radical, $R_1$ and $R_2$, being identical or different, each represents a hydrogen atom, a halogen atom, an alkyl radical containing from 1 to 8 carbon atoms, an alkoxy radical containing from 1 to 8 carbon atoms, a cyclohexyl radical, a phenoxy radical or a nitro radical, $R_3$ represents an alkoxy or alkylthio radical containing from 1 to 8 carbon atoms.

2. Derivatives answering to the general formula (I) of claim 1, as well as their salts, characterized in that in the said formula (I) R represents a methyl or phenyl radical, $R_1$ and $R_2$, being identical or different, each represents a hydrogen atom, a chlorine atom, a methyl, ethyl or methoxy radical, $R_3$ has the significance already indicated.

3. Derivatives answering to the formula (I) of claim 1, as well as their salts, characterized in that in the said formula (I) R represents a phenyl radical, $R_1$ represents a hydrogen atom, $R_2$ represents a hydrogen atom or an alkyl radical containing from 1 to 5 carbon atoms and $R_3$ represents an alkoxy radical containing from 1 to 5 carbon atoms.

4. Imidazo[1,2-a]quinoline answering to the formula (I) of claim 1, the name of which follows: (7-ethyl 5-methoxy-imidazo[1,2-a]quinolin-2-yl)phenyl methanone and its mesylate.

5. Imidazo[1,2-a]quinoline answering to formula (I) of claim 1, the name of which follows: (5-methoxy-imidazo[1,2-a]quinolin-2-yl)phenyl methanone.

6. Imidazo[1,2-a]quinoline answering to formula (I) of claim 1, the name of which follows: (5-isopropoxy-imidazo[1,2-a]quinolin-2-yl)phenyl methanone.

7. Imidazo[1,2-a]quinoline answering to formula (I) of claim 1, the name of which follows: (5-methylthio-imidazo[1,2-a]quinolin-2-yl)phenyl methanone.

8. Preparation process for products answering to the general formula (I), as well as their salts, characterized in that a product with the formula (II):

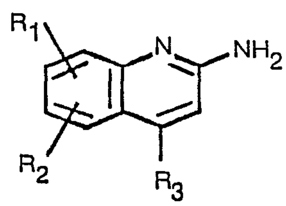

(II)

in which $R_1$, $R_2$ and $R_3$ have the significance already indicated, is made to react with a product with the formula (III):

$$X—CH_2—CO—CO—R \quad (III)$$

in which R has the significance already indicated and X represents a halogen atom, so as to obtain a product with the formula (IV):

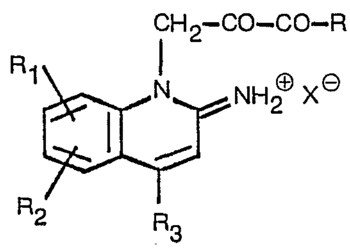

(IV)

in which R, $R_1$, $R_2$, $R_3$ and X have the significance already indicated, then the product with the formula (IV)

obtained is cyclised, so as to form the product with the general formula (I), which, if required, is salified.

9. Process according to claim 8, characterized in that:

— the reaction of the product with the formula (II) with the product with the formula (III) is carried out in an organic solvent such as dimethoxymethane;

— the cyclisation of the product with the formula (IV) is carried out by heating in an organic solvent such as ethanol;

— the reaction of the product with the formula (II) with the product with the formula (III) is continued to the product with the formula (I) without isolating the product with the formula (IV).

10. Preparation process for products such as those defined by the formula (I) of claim 1, answering to the formula ($I_a$):

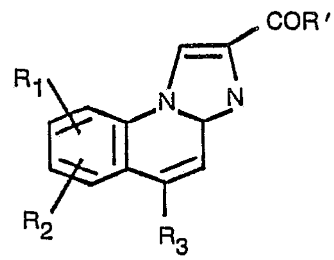

($I_a$)

in which R' represents a phenyl radical possibly substituted by one or more substituents chosen from the group composed of halogens, alkyl radicals containing from 1 to 5 carbon atoms and alkoxy radicals containing from 1 to 5 carbon atoms, or R' represents a thienyl radical or a pyridyl radical and $R_1$, $R_2$ and $R_3$ have the significance already indicated, as well as their salts, characterized in that a product with the formula (V):

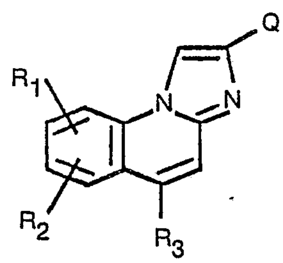

(V)

in which $R_1$, $R_2$ and $R_3$ have the significance already indicated and Q represents an esterified carboxyl group, is treated with a reducing agent so as to obtain a product with the formula (VI):

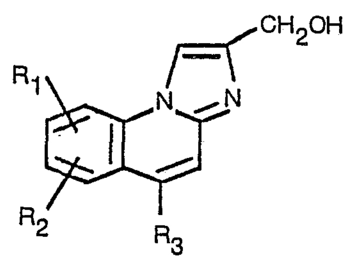

(VI)

in which $R_1$, $R_2$ and $R_3$ have the significance already indicated, which is treated with an oxidizing agent, so as to obtain a product with the formula (VII):

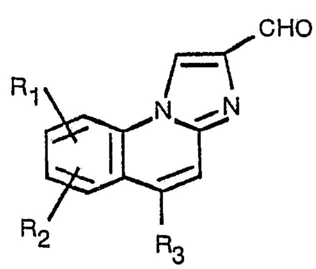

(VII)

in which $R_1$, $R_2$ and $R_3$ have the significance already indicated, which is made to react with a reagent with the formula (VIII):

R'—Y (VIII)

in which R′ has the significance already indicated and Y represents a chlorine, bromine or iodine atom, so as to obtain a product with the formula (IX):

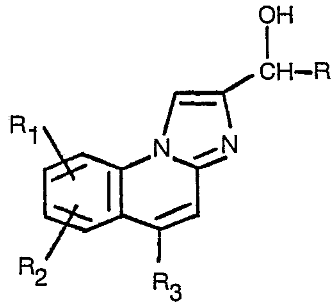

(IX)

in which $R_1$, $R_2$, $R_3$ and R′ have the significance already indicated, which is treated by an oxidizing agent, so as to obtain a product with the formula ($I_a$) which, if required, is salified.

11. Process according to claim 10, characterized in that:

— Q, in the product with the formula (V), represents an alkoxycarbonyl, preferably in which alkoxy contains from 1 to 3 carbon atoms, or aralkoxycarbonyl group;

— the reducing agent of the product with the formula (V) is lithium aluminium hydride;

— the oxidizing agent of products with the formulae (VI) and (IX) is manganese dioxide;

— the reaction of the product with the formula (VII) with the reagent with the formula (VIII) is caried out in anhydrous conditions, in an organic solvent, preferably tetrahydrofuran.

12. Medicaments, characterized in that they are composed of imidazo[1,2-a]quinolines as defined in claim 1, as well as by their salts of addition with pharmaceutically acceptable acids.

13. Medicaments, characterized in that they are composed of imidazo[1,2-a]quinolines as defined in claims 2 or 3, as well as by their salts of addition with pharmaceutically acceptable acids.

14. Medicaments, characterized in that they are composed of derivatives as defined in any one of the claims 4, 5, 6 or 7, as well as by the salts of addition with pharmaceutically acceptable acids of the said derivatives.

15. Pharmaceutical compositions, characterized in that they contain as active principle, at least one of the medicaments as defined in any one of the claims 12, 13 or 14.

16. As industrial products, products with the formula (IV):

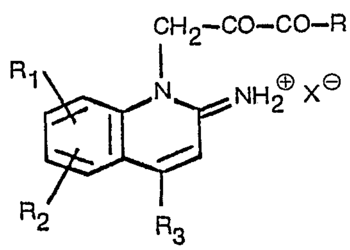

(IV)

as defined in claim 8.

17. As industrial products, products with the formula (X):

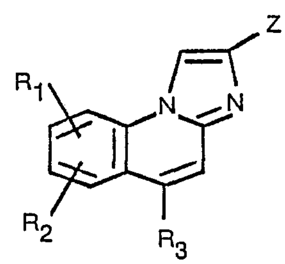

(X)

in which $R_1$, $R_2$ and $R_3$ are defined as in claim 1 and Z represents a group —CH O, —$CH_2OH$ or

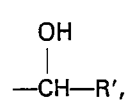

in which R′ is defined as in claim 10.

**Claims for the Contracting State: AT**

1. Process for preparing imidazo[1,2-a]quinolines answering to the general formula:

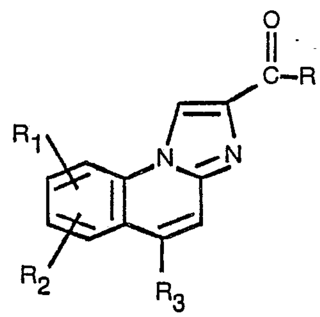

(I)

in which R represents an alkyl radical containing from 1 to 8 carbon atoms, a phenyl radical possibly substituted by one or more substituents chosen from the group constituted by the halogens, the alkyl radicals containing from 1 to 5 carbon atoms and the alkoxy radicals containing from 1 to 5 carbon atoms, or R represents a thienyl radical or a pyridyl radical, $R_1$ and $R_2$, being identical or different, each represents a hydrogen atom, a halogen atom, an alkyl radical containing from 1 to 8 carbon atoms, an alkoxy radical containing from 1 to 8 carbon atoms, a cyclohexyl radical, a phenoxy radical or a nitro radical, $R_3$ represents an alkoxy or alkylthio radical containing from 1 to 8 carbon atoms, as well as their salts, characterized in that a product with the formula (II):

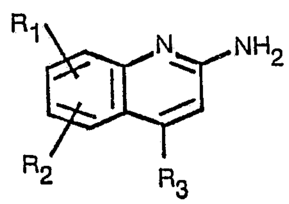

(II)

in which $R_1$, $R_2$ and $R_3$ have the significance already indicated, is made to react with a product with the formula (III):

$$X—CH_2—CO—CO—R \qquad (III)$$

in which R has the significance already indicated and X represents a halogen atom, so as to obtain a product with the formula (IV):

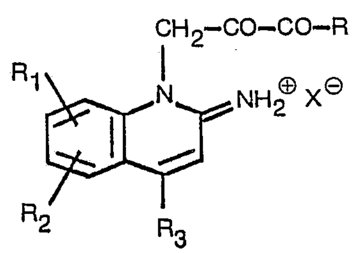

(IV)

in which R, $R_1$, $R_2$, $R_3$ and X have the significance already indicated, then the product with the formula (IV) obtained is cyclised, so as to form the product with the general formula (I), which, if required, is salified.

2. Process according to claim 1, characterized in that:

— the reaction of the product with the formula (II) with the product with the formula (III) is carried out in an organic solvent such as dimethoxymethane;

— the cyclisation of the product with the formula (IV) is carried out by heating in an organic solvent such as ethanol;

— the reaction of the product with the formula (II) with the product with the formula (III) is continued to the product with the formula (I) without isolating the product with the formula (IV).

3. Preparation process for products such as those defined by the formula (I) of claim 1, answering to the formula ($I_a$):

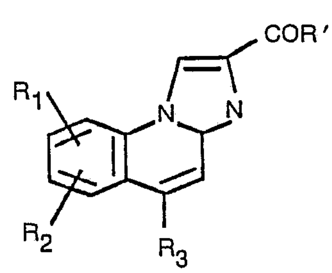

($I_a$)

in which R′ represents a phenyl radical possibly substituted by one or more substituents chosen from the group composed of halogens, alkyl radicals containing from 1 to 5 carbon atoms and alkoxy radicals containing from 1 to 5 carbon atoms, or R′ represents a thienyl radical or a pyridyl radical and $R_1$, $R_2$ and $R_3$ have the significance already indicated, as well as their salts, characterized in that a product with the formula (V):

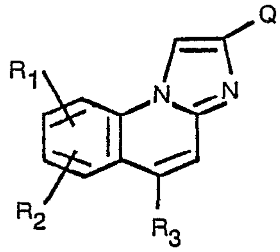

(V)

in which $R_1$, $R_2$ and $R_3$ have the significance already indicated and Q represents an esterified carboxyl group, is treated with a reducing agent so as to obtain a product with the formula (VI):

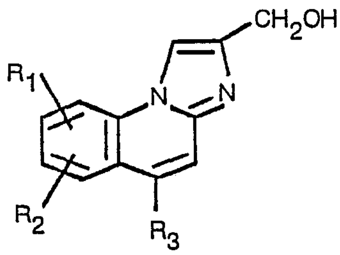

(VI)

in which $R_1$, $R_2$ and $R_3$ have the significance already indicated, which is treated with an oxidizing agent, so as to obtain a product with the formula (VII):

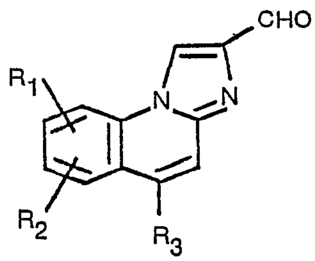

(VII)

in which $R_1$, $R_2$ and $R_3$ have the significance already indicated, which is made to react with a reagent with the formula (VIII):

$$R'—Y \qquad (VIII)$$

in which R′ has the significance already indicated and Y represents a chlorine, bromine or iodine atom, so as to obtain a product with the formula (IX):

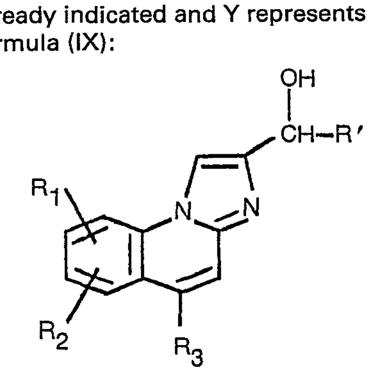

(IX)

in which $R_1$, $R_2$, $R_3$ and R′ have the significance already indicated, which is treated by an oxidizing agent, so as to obtain a product with the formula ($I_a$) which, if required, is salified.

4. Process according to claim 3, characterized in that:

— Q, in the product with the formula (V), represents an alkoxycarbonyl, preferably in which alkoxy contains from 1 to 3 carbon atoms, or aralkoxycarbonyl group;

— the reducing agent of the product with the formula (V) is lithium aluminium hydride;

— the oxidizing agent of the products with the formulae (VI) and (IX) is manganese dioxide;

— the reaction of the product with the formula (VII) with the reagent with the formula (VIII) is carried out in anhydrous conditions, in an organic solvent, preferably tetrahydrofuran.

5. Process according to any one of the claims 1 to 4, characterized in that derivatives with the formula I are prepared in which R represents a methyl or phenyl radical, $R_1$ and $R_2$, being identical or different, each represents a hydrogen atom, a chlorine atom, a methyl, ethyl or methoxy radical, $R_3$ has the significance already indicated.

6. Process according to any one of the claims 1 to 4, characterized in that derivatives of the formula I are prepared in which R represents a phenyl radical, $R_1$ represents a hydrogen atom, $R_2$ represents a hydrogen atom or an alkyl radical containing from 1 to 5 carbon atoms and $R_3$ represents an alkoxy radical containing from 1 to 5 carbon atoms.

7. Process according to any one of the claims 1 to 4, characterized in that there are prepared any one of the derivatives with the formula I, the names of which follow:

— (7-ethyl 5-methoxy-imidazo[1,2-a]quinolin-2-yl)phenyl methanone and its mesylate.
— (5-methoxy-imidazo[1,2-a]quinolin-2-yl)phenyl methanone.
— (5-isopropoxy-imidazo[1,2-a]quinolin-2-yl)phenyl methanone.
— (5-methylthio-imidazo[1,2-a]quinolin-2-yl)phenyl methanone.

**Patentansprüche für die Vertragsstaaten: BE CH DE FR IT LI LU NL SE**

1. Imidazo[1.2-a]chinoline und deren Salze, dadurch gekennzeichnet, daß sie der allgemeinen Formel

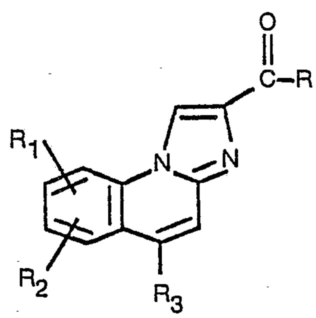

(I)

entsprechen, worin R einen Alkylrest mit 1 bis 8 Kohlenstoffatomen, einen Phenylrest, der gegebenenfalls durch einen oder mehrere Substituenten, ausgewählt unter den Halogenen, den Alkylresten mit 1 bis 5 Kohlenstoffatomen und den Alkoxyresten mit 1 bis 5 Kohlenstoffatomen, substituiert ist, bedeutet oder R einen Thienylrest oder einen Pyridylrest bedeutet; $R_1$ und $R_2$, die gleich oder verschieden sind, ein Wasserstoffatom, ein Halogenatom, einen Alkylrest mit 1 bis 8 Kohlenstoffatomen, einen Alkoxyrest mit 1 bis 8 Kohlenstoffatomen, einen Cyclohexylrest, einen Phenoxyrest oder eine Nitrogruppe bedeuten; $R_3$ einen Alkoxy- oder Alkylthiorest mit 1 bis 8 Kohlenstoffatomen bedeutet.

2. Derivate der Formel (I) gemäß Anspruch 1 sowie deren Salze, dadurch gekennzeichnet, daß in der Formel (I) R einen Methyl- oder Phenylrest bedeutet $R_1$ und $R_2$, die gleich oder verschieden sind, ein Wasserstoffatom, ein Chloratom, einen Methyl-, Ethyl- oder Methoxyrest bedeuten und $R_3$ die angegebene Bedeutung besitzt.

3. Derivate der Formel (I) gemäß Anspruch 1 sowie deren Salze, dadurch gekennzeichnet, daß in der Formel (I) R einen Phenylrest bedeutet, $R_1$ ein Wasserstoffatom bedeutet, $R_2$ ein Wasserstoffatom oder einen Alkylrest mit 1 bis 5 Kohlenstoffatomen bedeutet und $R_3$ einen Aloxyrest mit 1 bis 5 Kohlenstoffatomen bedeutet.

4. Imidazo[1.2-a]chinoline der Formel (I) gemäß Anspruch 1 mit der folgenden Bezeichnung: (7-Ethyl-5-methoxy-imidazo[1.2-a]chinolin-2-yl)-phenyl-methanon und sein Mesylat.

5. Imidazo[1.2-a]chinoline der Formel (I) gemäß Anspruch 1 mit der folgenden Bezeichnung: (5-Methoxy-imidazo[1.2-a]chinolin-2-yl)-phenyl-methanon.

6. Imidazo[1.2-a]chinoline der Formel (I) gemäß Anspruch 1 mit der folgenden Bezeichnung: (5-Isopropoxy-imidazo[1.2-a]chinolin-2-yl)-phenyl-methanon.

7. Imidazo[1.2-a]chinolin der Formel (I) gemäß Anspruch 1 mit der folgenden Bezeichnung: (5-Methylthio-imidazo[1.2-a]chinolin-2-yl)-phenyl-methanon.

8. Verfahren zur Herstellung der Produkte der allgemeinen Formel (I) sowie von deren Salzen, dadurch gekennzeichnet, daß man ein Produkt der Formel (II)

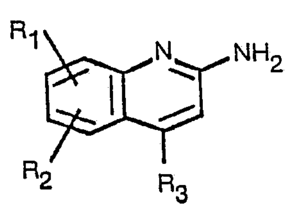

(II)

worin $R_1$, $R_2$ und $R_3$ die angegebene Bedeutung besitzen, mit einem Produkt der Formel (III)

$$X—CH_2—CO—CO—R \quad (III)$$

worin R die angegebene Bedeutung besitzt und X ein Halogenatom bedeutet, umsetzt, um ein Produkt der Formel (IV)

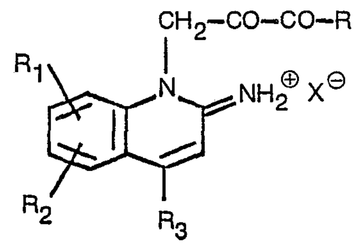

(IV)

zu erhalten, worin R, $R_1$, $R_2$, $R_3$ und X die angegebene Bedeutung besitzen, danach das erhaltene Produkt der Formel (IV) cyclisiert, um das Produkt der allgemeinen Formel (I) zu erhalten, das man gegebenenfalls in ein Salz überführt.

9. Verfahren gemäß Anspruch 8, dadurch gekennzeichnet, daß

die Umsetzung des Produkts der Formel (II) mit dem Produkt der Formel (III) in dem Medium eines organischen Lösungsmittels, wie Dimethoxymethan, durchgeführt wird;

die Cyclisierung des Produkts der Formel (IV) durch Erwärmen in dem Medium eines organischen Lösungsmittels, wie Ethanol, durchgeführt wird;

die Umsetzung des Produkts der Formel (II) mit dem Produkt der Formel (III) bis zum Produkt der Formel (I) ohne Isolierung des Produkts der Formel (IV) fortgesetzt wird.

10. Verfahren zur Herstellung der Produkte der Formel (I) gemäß Anspruch 1 der Formel ($I_a$)

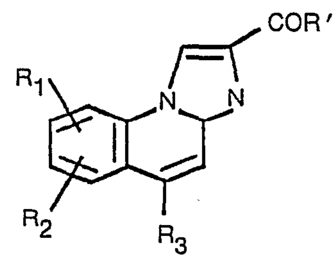

($I_a$)

worin R' einen Phenylrest bedeutet, der gegebenenfalls durch einen oder mehrere Substituenten, ausgewählt unter den Halogenen, den Alkylresten mit 1 bis 5 Kohlenstoffatomen und den Alkoxyresten mit 1 bis 5 Kohlenstoffatomen, substituiert ist, oder R' einen Thienyl- oder Pyridylrest bedeutet und $R_1$, $R_2$ und $R_3$ die angegebene Bedeutung besitzen, sowie von deren Salzen, dadurch gekennzeichnet, daß man ein Produkt der Formel (V)

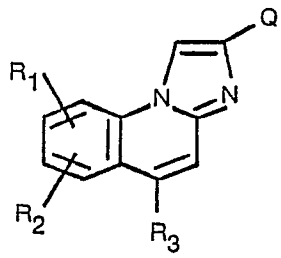

(V)

worin $R_1$, $R_2$ und $R_3$ die angegebene Bedeutung besitzen und Q eine veresterte Carboxylgruppe bedeutet, mit einem Reduktionsmittel behandelt, um ein Produkt der Formel (VI)

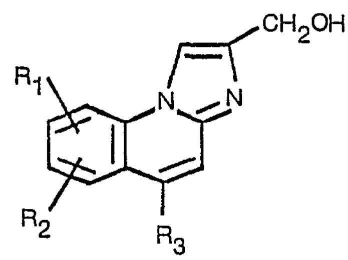

(VI)

zu erhalten, worin $R_1$, $R_2$ und $R_3$ die angegebene Bedeutung besitzen, das man mit einem Oxidationsmittel behandelt, um ein Produkt der Formel (VII)

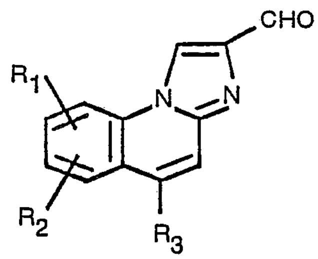

(VII)

zu erhalten, worin $R_1$, $R_2$ und $R_3$ die angegebene Bedeutung besitzen, das man mit einem Reagens der Formel (VIII)

$$R'—Y \qquad (VIII)$$

umsetzt, worin R' die angegebene Bedeutung besitzt und Y ein Chlor-, Brom- oder Jodatom bedeutet, um ein Produkt der Formel (IX)

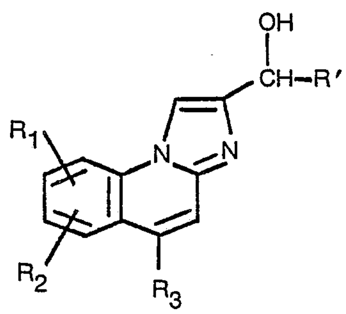

(IX)

zu erhalten, worin $R_1$, $R_2$, $R_3$ und R' die angegebene Bedeutung besitzen, das man mit einem Oxidationsmittel behandelt, um ein Produkt der Formel ($I_a$) zu erhalten, das man gewünschtenfalls in ein Salz überführt.

11. Verfahren gemäß Anspruch 10, dadurch gekennzeichnet, daß

Q in dem Produkt der Formel (V) eine Alkoxycarbonylgruppe, vorzugsweise eine solche, in der die Alkoxygruppe 1 bis 3 Kohlenstoffatomen umfaßt, oder eine Aralkoxycarbonylgruppe bedeutet;

das Reduktionsmittel für das Produkt der Formel (V) das Lithiumaluminiumhydrid ist;

das Oxidationsmittel für die Produkte der Formeln (VI) und (IX) das Mangandioxid ist;

die Umsetzung des Produktes der Formel (VII) mit dem Reagens der Formel (VIII) unter wasserfreien Bedingungen in einem organischen Lösungsmittel, vorzugsweise Tetrahydrofuran, durchgeführt wird.

12. Arzneimittel, dadurch gekennzeichnet, daß sie aus den Imidazo[1.2-a]chinolinen gemäß Anspruch 1 sowie aus deren Additionssalzen mit pharmazeutisch verträglichen Säuren bestehen.

13. Arzneimittel, dadurch gekennzeichnet, daß sie aus den Imidazo[1.2-a]chinolinen gemäß Anspruch 2 oder 3 sowie aus deren Additionssalzen mit pharmazeutisch verträglichen Säuren bestehen.

14. Arzneimittel, dadurch gekennzeichnet, daß sie aus den Derivaten gemäß einem der Ansprüche 4, 5, 6 oder 7 sowie aus deren Additionssalzen mit pharmazeutisch verträglichen Säuren bestehen.

15. Pharmazeutische Zusammensetzungen, dadurch gekennzeichnet, daß sie als Wirkstoff zumindest eines der Arzneimittel gemäß einem der Ansprüche 12, 13 oder 14 enthalten.

16. Als industrielle Produkte die Produkte der Formel (IV)

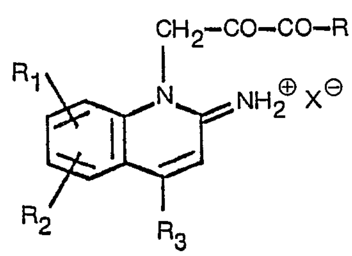

(IV)

gemäß Anspruch 8.

17. Als industrielle Produkte die Produkte der Formel (X)

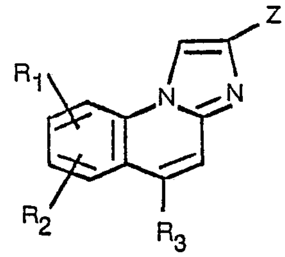

(X)

worin $R_1$, $R_2$ und $R_3$ wie in Anspruch 1 definiert sind und Z eine Gruppe $—CHO$, $—CH_2OH$ oder

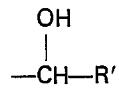

bedeutet, worin R′ die in Anspruch 10 angegebene Bedeutung besitzt.

**Patentansprüche für den Vertragsstaat: AT**

1. Verfahren zur Herstellung von Imidazo[1.2-a]chinolinen der allgemeinen Formel

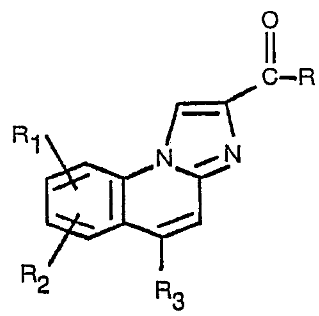

(I)

worin R einen Alkylrest mit 1 bis 8 Kohlenstoffatomen oder einen Phenylrest bedeutet, der gegebenenfalls durch einen oder mehrere Substituenten, ausgewählt unter den Halogenen, den Alkylresten mit 1 bis 5 Kohlenstoffatomen und den Alkoxyresten mit 1 bis 5 Kohlenstoffatomen, substituiert ist, oder R einen Thienyl- oder einen Pyridylrest bedeutet, $R_1$ und $R_2$, die gleich oder verschieden sind, ein Wasserstoffatom, ein Halogenatom, einen Alkylrest mit 1 bis 8 Kohlenstoffatomen, einen Alkoxyrest mit 1 bis 8 Kohlenstoffatomen, einen Cyclohexylrest, einen Phenoxyrest oder eine Nitrogruppe bedeuten und $R_3$ einen Alkoxy- oder Alkylthiorest mit 1 bis 8 Kohlenstoffatomen bedeutet, sowie von deren Salzen, dadurch gekennzeichnet, daß man ein Produkt der Formel (II)

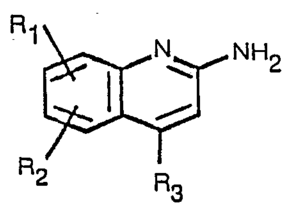

(II)

worin $R_1$, $R_2$ und $R_3$ die angegebene Bedeutung besitzen, mit einem Produkt der Formel (III)

$$X—CH_2—CO—CO—R \qquad (III)$$

worin R die angegebene Bedeutung besitzt und X ein Halogenatom bedeutet, umsetzt, um ein Produkt der Formel (IV)

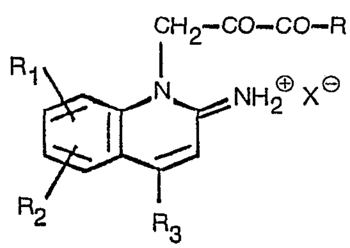

(IV)

zu erhalten, worin R, $R_1$, $R_2$, $R_3$ und X die angegebene Bedeutung besitzen, und danach das erhaltene Produkt der Formel (IV) cyclisiert, um das Produkt der allgemeinen Formel (I) zu bilden, das man gegebenenfalls in ein Salz überführt.

2. Verfahren gemäß Anspruch 1, dadurch gekennzeichnet, daß

die Umsetzung des Produkts der Formel (II) mit dem produkt der Formel (III) in dem Medium eines organischen Lösungsmittels, wie Dimethoxymethan, durchgeführt wird;

die Cyclisierung des Produkts der Formel (IV) durch Erwärmen in dem Medium eines organischen Lösungsmittels, wie Ethanol, erfolgt;

die Umsetzung des Produktes der Formel (II) mit dem Produkt der Formel (III) bis zu dem Produkt der Formel (I) ohne Isolierung des Produkts der Formel (IV) fortgesetzt wird.

3. Verfahren zur Herstellung der Produkte der Formel (I) gemäß Anspruch 1 mit der Formel ($I_a$)

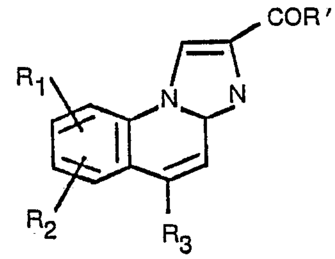

($I_a$)

worin R' einen Phenylrest bedeutet, der gegebenenfalls durch einen oder mehrere Substituenten, ausgewählt unter den Halogenen, den Alkylresten mit 1 bis 5 Kohlenstoffatomen und den Alkoxyresten mit 1 bis 5 Kohlenstoffatomen, substituiert ist, oder R' einen Thienyl- oder einen Pyridylrest bedeutet und $R_1$, $R_2$ und $R_3$ die angegebene bedeutung besitzen, sowie von deren Salzen, dadurch gekennzeichnet, daß man ein Produkt der Formel (V)

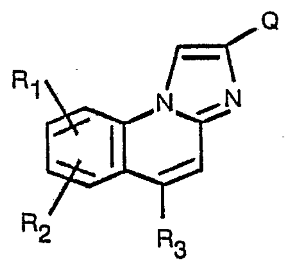

(V)

worin $R_1$, $R_2$ und $R_3$ die angegebene bedeutung besitzen und Q eine veresterte Carboxylgruppe bedeutet, mit einem Reduktionsmittel behandelt, um ein Produkt der Formel (VI)

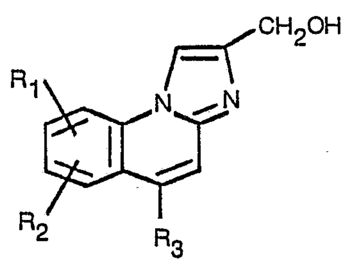

(VI)

zu erhalten, worin $R_1$, $R_2$ und $R_3$ die angegebene Bedeutung besitzen, das man mit einem Oxidationsmittel behandelt, um ein Produkt der Formel (VII)

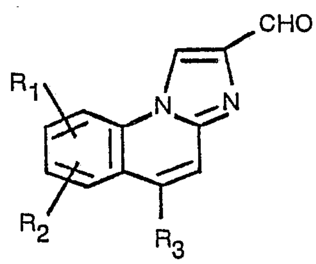

(VII)

zu erhalten, worin $R_1$, $R_2$ und $R_3$ die angegebene Bedeutung besitzen, das man mit einem Reagens der Formel (VIII)

$$R'{-}Y \qquad (VIII)$$

behandelt, worin R' die angegebene Bedeutung besitzt und Y ein Chlor-, Brom oder Jodatom bedeutet, um ein Produkt der Formel (IX)

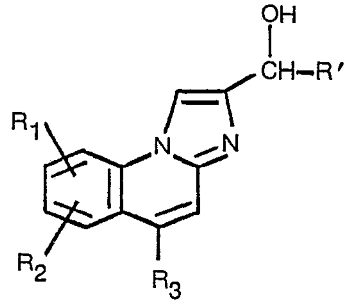

(IX)

zu erhalten, worin $R_1$, $R_2$, $R_3$ und R' die angegebene Bedeutung besitzen, das man mit einem Oxidationsmittel behandelt, um ein Produkt der Formel ($I_a$) zu erhalten, das man gegebenenfalls in ein Salz überführt.

4. Verfahren gemäß Anspruch 3, dadurch gekennzeichnet, daß

Q in dem Produkt der Formel (V) eine Alkoxycarbonylgruppe, vorzugsweise eine solche, in der die Alkoxygruppe 1 bis 3 Kohlenstoffatome umfaßt, oder eine Aralkoxycarbonylgruppe bedeutet;

das Reduktionsmittel für das Produkt der Formel (V) das Lithiumaluminiumhydrid ist;

das Oxidationsmittel für die Produkte der Formeln (VI) und (IX) Mangandioxid ist;

die Umsetzung des Produktes der Formel (VII) mit dem Reagens der Formel (VIII) unter wasserfreien Bedingungen in einem organischen Lösungsmittel, vorzugsweise Tetrahydrofuran, durchgeführt wird.

5. Verfahren gemäß einem der Ansprüche 1 bis 4, dadurch gekennzeichnet, daß man die Derivate der Formel (I) herstellt, worin R einen Methyl- oder Phenylrest bedeutet, $R_1$ und $R_2$, die gleich oder verschieden sind, ein Wasserstoffatom, ein Chloratom, einen Methyl-, Ethyl- oder Methoxyrest bedeuten und $R_3$ die angegebene Bedeutung besitzt.

6. Verfahren gemäß einem der Ansprüche 1 bis 4, dadurch gekennzeichnet, daß man die Derivate der Formel (I) herstellt, worin R einen Phenylrest bedeutet, $R_1$ ein Wasserstoffatom bedeutet, $R_2$ ein Wasserstoffatom oder einen Alkylrest mit 1 bis 5 Kohlenstoffatomen bedeutet und $R_3$ einen Alkoxyrest mit 1 bis 5 Kohlenstoffatomen bedeutet.

7. Verfahren gemäß einem der Ansprüche 1 bis 4, dadurch gekennzeichnet, daß man Derivate der Formel (I) mit einer der folgenden Bezeichnungen herstellt:

(7-Ethyl-5-methoxy-imidazo[1.2-a]chinolin-2-yl)-phenyl-methanon und sein Mesylat;

(5-Methoxy-imidazo[1.2-a]chinolin-2-yl)-phenyl-methanon;

(5-Isopropoxy-imidazo[1.2-a]chinolin-2-yl)-phenyl-methanon;

(5-Methylthio-imidazo[1.2-a]chinolin-2-yl)-phenyl-methanon.